Europäisches Patentamt

European Patent Office

Office européen des brevets

(11) Publication number: **0 357 892 B1**

## EUROPEAN PATENT SPECIFICATION

(45) Date of publication of patent specification: **16.12.92**   (51) Int. Cl.⁵: **A61F 13/38**, A61B 10/00

(21) Application number: **89110921.7**

(22) Date of filing: **16.06.89**

(54) **Swab for collection of biological samples.**

(30) Priority: **13.07.88 US 218714**

(43) Date of publication of application:
**14.03.90 Bulletin 90/11**

(45) Publication of the grant of the patent:
**16.12.92 Bulletin 92/51**

(84) Designated Contracting States:
**AT BE CH DE ES FR GB GR IT LI LU NL SE**

(56) References cited:
DE-C- 472 556          FR-A- 2 076 769
US-A- 2 170 222        US-A- 2 362 704
US-A- 2 491 274        US-A- 3 352 307
US-A- 4 401 130        US-A- 4 492 305
US-A- 4 746 238

(73) Proprietor: **Becton Dickinson and Company
One Becton Drive
Franklin Lakes New Jersey 07417-1880(US)**

(72) Inventor: **Kachigian, Corinne S.
Six Plum Court
Flemington New Jersey(US)**

(74) Representative: **Selting, Günther, Dipl.-Ing. et al
Patentanwälte von Kreisler, Selting, Werner
Deichmannhaus am Hauptbahnhof
W-5000 Köln 1(DE)**

## Description

The present invention relates to swabs for collecting biological samples and more particularly concerns a swab having improved handle features and a swabbing tip made of closed cell foam.

Traditionally, testing for microorganisms such as Streptococcus has been performed by, for example, taking a sample from the patient's throat using a soft tipped stab in order to accumulate as large a sample of the suspected organism as reasonably possible. The organism was then placed in a culture medium and grown until sufficient quantities existed for identification. Swabs used to gather microorganism samples have been made of fibrous materials such as cotton, polyester and rayon, and open pore or open cell foam as taught in US-A-3,724,018. The more microorganisms that could be collected the faster the colonies could be grown to sizes necessary to reach a conclusion regarding the presence of specific microorganisms. Accordingly, many swab designs and related accessories have been developed to assure a reasonable sample size and to protect the microorganisms in the time period between sampling and culturing. For example, US-A-4,492,305 teaches a culture collection system which attempts to keep the culture alive for a period of time until it is tested. This document teaches a culture collecting swab and a sealed glass ampoule with a resilient closure member positioned at the stem of the swab. After culture collection the ampoule is broken open, along a score line, and the tip end of the swab is inserted into the ampoule through the open end into culture-sustaining media wherein further penetration causes the resilient closure to seal the opening thus defining a culture sustaining environment for the swab and the collected microorganisms until testing.

Although traditional testing and culture growth methods have evolved into more efficient systems there is still a substantial time delay between the specimen collection and the actual determination. When dealing with life threatening or serious health hazards these time delays still present impediments to timely diagnosis and treatment. Substantial improvements in health care have been achieved by newly developed improved testing procedures which result in a determination in hours and in sometimes minutes after sample collection. These short duration tests do not rely on the collection and growth of microorganisms but look to identify chemical characteristics of the suspected biological substance such as an antigen which is associated with, for example, Streptococcus. Tests along these lines seek only to gather portions of the Streptococci, the antigen, and expose this biological sample to an antibody which is known to attach itself to the specific antigen. Accordingly, if the antigen is present the antibody will attach to it and a determination may be made. The prior art has taught attaching radioisotopes to antibodies so that if the antibody attaches to the antigen there will be a concentration of radioactive material at the attaching sites. This type of testing is known as a technique of a radioimmunoassay (RIA). Obviously, unattached antibodies must be removed so that only the combined antibody-antigen groups will remain. Antibodies have also been coupled with fluorochromes which are responsive to light and to enzymes which can react with additional substances to produce color changes and more recently to liposomes which contain color dyes.

US-A-4,618,576 teaches a test that can be performed in less than seventy minutes to determine the presence of Group A Streptococcus. This document teaches the use of a fiber swab on the end of a stick for swabbing the infected area to collect antigen in its fibers. Subsequently, the swab is dipped in a solution of extraction reagent containing enzymes which facilitate the release of the Streptococcus A antigen from the swab. Finally, an indicator reagent is provided with antibodies that are specifically reactive to the Streptococcus A antigen.

As technology allows the development of tests that are faster, it is more important to take the sample and deliver it to the test site in a timely manner. It may no longer be practical to have a fibrous swab or a tip containing interconnected cavities which trap the biological specimen thus requiring time to extract it from the interior of the swab or special solutions to promote the removal of the biological substance from the swab. Faster tests may be complemented with improved swab structures which allow the effective collection of a biological sample and its rapid transfer to the test system.

Also, traditional swabs having cotton or other fiber tips on rigid rod-like shaft portions made of wood or plastic present potential problems due to the underlying sharp edges on rigid shaft portions which may irritate or injure the collection area if the sample is not properly and carefully taken. This potential for irritation is caused to some extent by the inability of the soft fibrous tip of the swab to provide complete protection from the sharp edge of the rod-like wood or plastic shaft. Also, the straight shaft structure is not necessarily designed to be anatomically correct with respect to the portion of the patient body wherein the sample is being taken or with respect to the sample taker's hand. US-A-3,871,375 provides an improved structure wherein the open cell tip and the shaft portion of the swab are molded in the same cavity so that the blended region joining the central shaft and the soft, resilient tip merges gradually from the low porosity more rigid shaft portion to the high porosity soft resilient tip so that there is no sharp ended "stick" presented to the patient's body.

US-A-4 401 130 discloses a swab having an open cell polyester urethane foam body fastened to a handle extending into a bore in the body. The handle is maintained by adhesive in the bore and the bore has a cushioned bottom to prevent the handle from penetrating therethrough during use. Also this open cell swab tip requires time to extract therefrom the biological specimen from the interior thereof.

Although the prior art has addressed improved apparatus and tests for the taking of biological samples and the subsequent transfer and testing of these samples, it is the object of the present invention to provide a simple, straight-forward, reliable, easily fabricated swab for collection of biological samples which provides for easier removal of the biological sample from the swabbing tip and improved structure, with respect to human factors, to improve the ease of sample gathering while minimizing the potential for irritation to the patient.

The invention is defined in claim 1. The dependent claims define preferred embodiments of the invention which is described hereinafter in detail with reference to the accompanying drawings.

Fig. 1 is a front elevation view of the preferred swab for collecting biological samples of the present invention;

Fig. 2 is a side elevation view of the swab for collecting biological samples of Fig. 1;

Fig. 3 is a rear elevation view of the swab for collecting biological samples of Fig. 1;

Fig. 4 is an enlarged partial cross-sectional view of the swab of Fig. 3 taken along line 4-4;

Fig. 5 is an enlarged cross-sectional view of the swab of Fig. 3 taken along line 5-5;

Fig. 6 is an enlarged cross-sectional view of the swab of Fig. 3 taken along line 6-6;

Fig. 7 is a partial side elevational view illustrating the swabbing tip of an alternative embodiment of the present invention;

Fig. 8 is a front elevation view illustrating still another alternative embodiment of the present invention; and

Fig. 9 is a side elevation view of the swab for collecting biological samples of Fig. 8.

While this invention is satisfied by embodiments in many different forms, there is shown in the drawings and will herein be described in detail preferred embodiments of the invention with the understanding that the present disclosure is to be considered as exemplary of the principles of the invention and not intended to limit the invention to the embodiments illustrated. The scope of the invention shall be measured by the appended claims and their equivalents.

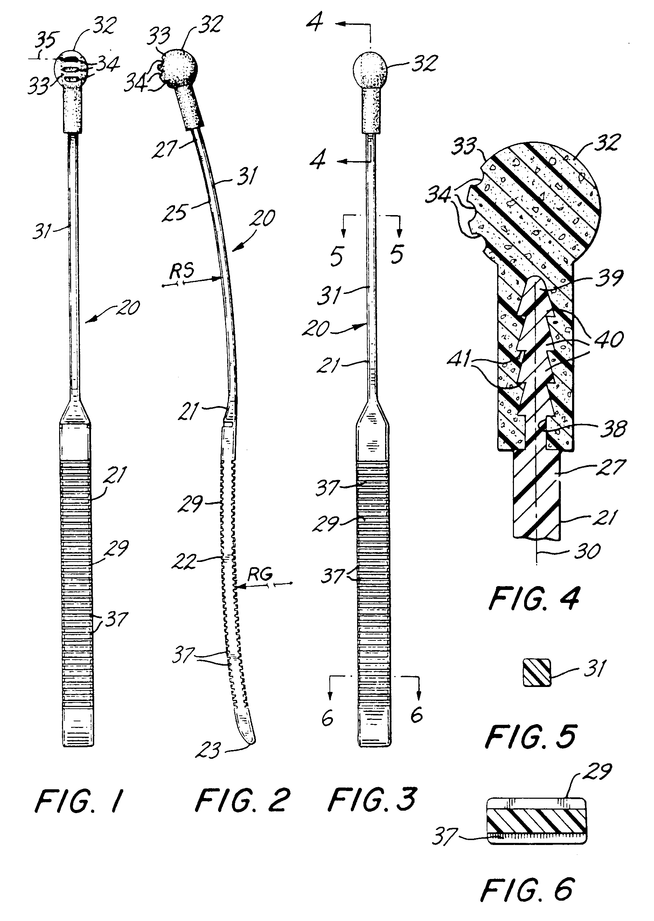

Adverting to Figs. 1 through 6, a preferred embodiment of the swab for collection of biological samples 20 includes a handle 21 having a proximal portion 22 including a proximal end 23 and a distal portion 25 including a distal end 27. For the purposes of the description of the present invention, the term "distal" is meant to refer to that end of the element which is furthest from the person holding the swab, whereas the term "proximal" is meant to refer to the end of the element closest to the holder of the swab.

Handle 21 includes a gripping portion 29 at the proximal portion and a shaft portion 31 at the distal portion. A feature of this embodiment is that gripping portion 29 is more rigid than shaft portion 31.

A swabbing tip 32 is provided at distal end 27 for contacting and collecting a biological sample. Swabbing tip 32 is formed of closed cell foam and is softer and more resilient than handle 21. It is preferred that the swabbing tip includes a convex surface for collection of biological samples. The convex shape is especially desirable when taking samples from body cavities such as a patient's throat wherein the rounded convex surface of the swabbing tip tends to be less irritating. However, since closed cell foam is used, the sample will remain substantially on the outside surface 33 of the swabbing tip. It is also desirable to have some form of surface discontinuity on the outside surface to facilitate the removal of the biological sample. In this preferred embodiment, the convex surface of the swabbing tip is spherically shaped and this surface includes a discontinuity for facilitating biological sample gathering in the form of concave grooves 34 having major axes running along their longer dimension. It is desirable, but not necessary, to orient these axes at an angle of between 45° and 90° with respect to a longitudinal axis 30 of the distal end of the handle. In this preferred embodiment, these axes are at an angle of about 90° with respect to longitudinal axis 30. Although the swabbing tip is convexly shaped and resilient to facilitate patient comfort the discontinuities provide a specific area on the surface of the swabbing tip which is more efficient for the purposes of gathering the biological sample.

In this preferred embodiment gripping portion 29 is curvalinearly shaped having a radius of curvature RG, as illustrated in Fig. 2. Also, the shaft portion 31 is curvalinearly shaped having a radius of curvature RS, as best illustrated in Fig. 2. In this preferred embodiment the origin of the radius of curvature for the gripping portion is opposed from the origin of the radius of curvature of the shaft portion so that the shaft portion appears convexly shaped when viewed from the origin of the radius of curvature of the gripping portion. In this preferred embodiment, a plane containing the shaft portion and the radius of curvature for the shaft portion is coextensive with a plane containing the gripping portion and the radius of curvature of the gripping portion. The curvalinearly shaped gripping portion and shaft portion are preferred but not a requirement of the invention. The curved gripping portion facilitates holding the handle while the reduced cross-section and curvalinear shape of the shaft portion provide a resiliency to minimize inadvertent irritation to the patient and to make it easier for the person taking the sample to access interior surfaces of the patient's body which are not in a straight line of sight from the exterior of the patient's body. The soft resilient swabbing tip and the resilient shaft portion are important features for controlling the pressure applied when taking the biological sample and minimizing inadvertent irritation to the patient.

Another feature of this preferred embodiment is means for facilitating holding the gripping portion and moving the swabbing tip while collecting a biological sample. In this preferred embodiment, this means includes external channels 37 on the gripping portion. Channels 37 are desirably oriented so that they extend across the longitudinal axis of the gripping portion. It will be apparent to one skilled in the art that numerous surface configurations can be used advantageously to facilitate holding the gripping portion for better control over the swabbing tip. These structures are within the purview of the instant invention and include, but are not limited to, parallel grooves or channels, a knurled surface having crossing grooves, a roughened surface to increase the coefficient friction and a coating over the gripping portion which is softer and more resilient to facilitate improved gripping.

In addition, the closed cell structure of the swabbing tip assures that the biological sample will remain substantially at the surface of the swabbing tip and in the discontinuities which are provided for holding the biological sample so that the task of removing the biological sample from the tip will be greatly simplified and additional steps or subjecting the tip to intermediate mixtures such as the use of an aqueous extraction reagent, as taught by Rosenstein et al. in U.S. Patent No. 4,618,576, may be eliminated. An immunoassay test for determination of the presence of a specific biological substance cannot take place in minutes if substantially more time is required to remove the biological sample from the sampling device. The use of the closed cell foam to keep the biological sample at the surface of the swabbing tip and the use of the discontinuities to facilitate sample gathering is a clear and substantial advantage over prior art swabs.

In this preferred embodiment swabbing tip 32 includes a substantially cylindrical, closed end, passageway 38 which engages engaging structure 39 at distal end 27 of handle 21 for the purpose of securing swabbing tip 32 to handle 21. In this preferred embodiment engaging structure 39 includes a series of frusto-conically shaped segments 40 oriented so that the swabbing tip may easily slip over the engaging structure with lower edges 41 of the frusto-conically shaped segments resisting removal of the swabbing tip from the handle. The above-described structure for holding swabbing tip 32 onto handle 21 is preferred because it can eliminate the need for adhesives and other attachment means. It will be apparent to one skilled in the art that there are numerous means and structures for securing a closed cell foam swabbing tip to a more rigid handle structure including, but not limited to, adhesives, ultrasonic welding, complimentary engaging structure, external clamps and the like with the above-described structure being exemplary of these many possibilities.

In use the swab for collecting biological samples of the present invention is used in conjunction with immunoassay type tests which are designed to provide diagnostic information in a time frame of minutes rather than hours or days. These tests do not rely on the culturing of bacteria samples but upon a chemical reaction with a portion of the biological substance which is thought to be present, such as the antigen from Streptococcus bacteria, as described hereinabove. Accordingly, as will be described in more detail hereinafter, it is desirable to provide the swab in a package so that the swab is sterile at the time of use. The medical practitioner holds the swab of the present invention by gripping portion 29 and directs the swabbing tip toward the surface or area wherein the sample is to be taken, for example, the patient's throat. The curvature of the gripping portion and the shaft portion of the preferred embodiment of the swab for collection of biological samples of the present invention offers advantages in that it is more easily gripped and the curved shaft portion allows access to surfaces that may not be in the direct line of sights or which are difficult to access with a straight shaft-type handle of prior art devices. Also the relative resilience of the shaft portion and the convex surface of the swabbing tip minimize the possibility of irritation to the patient during the sample gathering procedure. The swab of the present invention can be positioned so that the swabbing tip is within the patient's throat and the swabbing tip maneuvered to contact the areas of suspected infection by moving the swabbing tip inwardly and outwardly in a direction perpendicular to the grooves in the swabbing tip to facilitate maximizing the quantity of sample gathered. The swabbing tip may then be removed from the patient's throat so that the collected sample may be transferred to a test device for subsequent analysis.

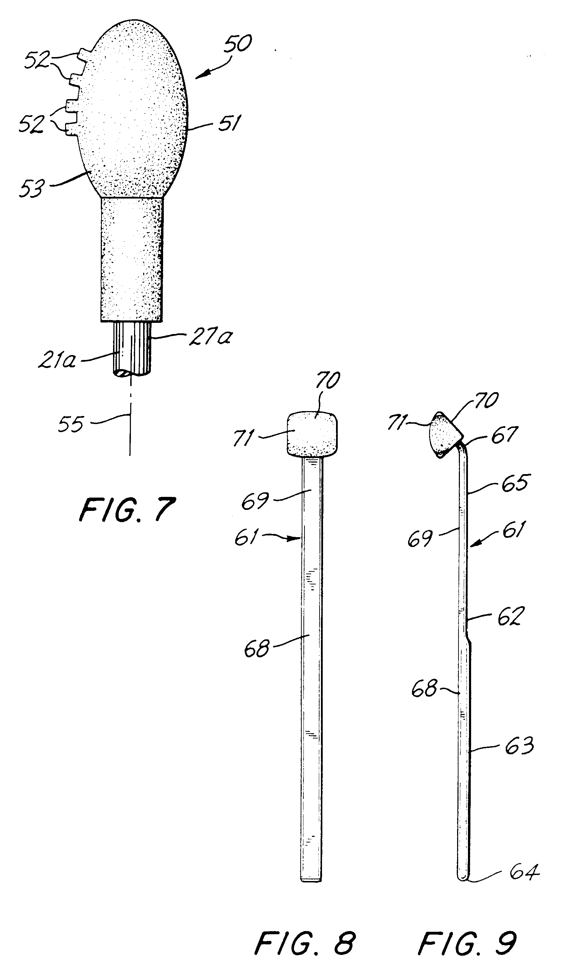

Referring now to Fig. 7, an alternative swab for the collection of biological samples 50 of the present invention includes components which are substantially identical to the components of the embodiment of Figs. 1-6. Accordingly, similar components performing similar functions will be numbered identically to those components in the embodiment of Figs. 1-6, except that the suffix "a" will be used to identify the components of Fig. 7. This alternative swab for the collection of biological samples includes a handle 21a having a distal end 27a and a proximal end (not shown). A swabbing tip is provided at distal end 27a for contacting and collecting a biological sample. Swabbing tip 51 is formed of closed cell foam and is softer and more resilient than handle 21a. It is preferred, but not necessary, that the swabbing tip have a convex surface for the collection of biological samples. In this embodiment, convex surface 53 of the swabbing tip is ellipsoidally shaped and this surface includes a discontinuity for facilitating biological sample gathering in the form of a plurality of raised protuberances 52 projecting outwardly from convex surface 53. The raised protuberances have major axes running along their longer dimension which in this embodiment places the major axes at an angle of about 90° with respect to the longitudinal axis 55 of distal end 27a. In other structural aspects the handle of the swab for the collection of biological samples of Fig. 7 is substantially similar to the handle of the embodiment of Figs. 1-6.

Referring now to Figs. 8 and 9 wherein an alternative swab for the collection of biological samples 61 is illustrated. Swab 61 includes a handle 62 having a proximal portion 63 including a proximal end 64 and a distal portion 65 having a distal end 67. Handle 62 also includes gripping portion 68 at the proximal portion and the shaft portion 69. In this embodiment the gripping portion 68 is more rigid than shaft portion 69.

A swabbing tip 70 is provided at distal end 67 for contacting and collecting a biological sample. Swabbing tip 70 is formed of closed cell foam and is softer and more resilient than handle 21. It is preferred that the stabbing tip have a convex surface 71 for the collection of biological samples.

A wide variety of rigid materials are suitable for the manufacture of the handle of the present invention including metal, wood, and plastic materials with thermoplastics being preferred because of the availability of manufacturing processes, such as injection molding, which allow the easy formation of a handle having the various shapes and surfaces described hereinabove. Thermoplastic materials such as polystyrene, polypropylene and polyethylene are preferred.

A wide variety of closed cell foam materials are suitable for the formation of the swabbing tip of the present invention with closed cell polyethylene foam being preferred. Such a closed cell polyethylene foam is available in formed parts from Illbruck/USA of Minneapolis, Minnesota 55412, USA under the description of N-200-A semi-rigid closed cell crosslinked polyethylene foam having a density of two (2) pounds per cubic foot (0.032 gram/cm$^3$). Closed cell foam may also be purchased in rectangular shapes and formed to the desired shape by the swab manufacturer. An important feature of the present swab for the collection of biological samples is the use of closed cell foam producing the benefits described hereinabove.

It can be seen that the present swab for the collection of biological samples provides a simple, straight-forward, reliable, easily fabricated swab for the collection of biological samples which provides for easy removal of the biological sample from the swabbing tip and improved structure, with respect to human factors, to improve the ease of sample gathering while minimizing the potential for irritation to the patient.

## Claims

1. A swab for collecting biological samples comprising:

   an elongate handle (21) having a proximal portion (22) including a proximal end (23) and a distal portion (25) including a distal end (27), said handle including a shaft portion (31) at said distal portion (25) and a gripping portion (29) at said proximal portion (22); and

   a swabbing tip (32) at said distal end (27) for contacting and collecting a biological sample, said swabbing tip (32) being formed of polymeric foam, said swabbing tip being softer and more resilient than said handle,
   **characterized in that**
   said polymeric foam of said swabbing tip (32) is a closed cell polymeric foam and said swabbing tip includes surface discontinuity means for facilitating biological sample gathering.

2. The swab for collecting biological samples of claim 1 wherein said swabbing tip (32) is sterile.

3. The swab for collecting biological samples of claim 1 or 2 wherein said surface discontinuity means includes at least one concave groove (34) in the surface of said swabbing tip (32).

4. The swab for collecting biological samples of claim 1 or 2 wherein said surface discontinuity means includes at least one protuberance (52) projecting outwardly from the surface of said swabbing tip (32).

5. The swab for collecting biological samples of one of claims 1-4 wherein said gripping portion (29) is more rigid than said shaft portion (31).

6. The swab for collecting biological samples of one of claims 1-5 wherein said shaft portion (31) is curvalinearly shaped.

7. The swab for collecting biological samples of one of claims 1-6 wherein said gripping portion (29) is curvalinearly shaped.

8. The swab for collecting biological samples of one of claims 1-5 wherein said shaft portion (31) is curvalinearly shaped and said gripping portion (29) is curvalinearly shaped wherein the origin of the radius (RS) of curvature of said shaft portion (31) is opposed to the origin of the radius (RG) of curvature of said gripping portion (29).

9. The swab for collecting biological samples of claim 1 wherein said swabbing tip (32) is made of closed cell polyethylene foam.

10. The swab for collecting biological samples of claim 9 wherein said foam has a density of substantially about two pounds per cubic foot (32 kilograms per cubic meter).

**Patentansprüche**

1. Tupfer zum Sammeln von biologischen Proben, mit

einem länglichen Handgriff (21) mit einem ein proximales Ende (23) aufweisenden proximalen Teil (22) und einem ein distales Ende (27) aufweisenden distalen Teil (25), wobei der Handgriff an dem distalen Teil (25) einen Schaftteil (31) und an dem proximalen Teil (22) einen Griffteil (29) aufweist; und

einem an dem distalen Ende (27) befindlichen Abtupfende (32) zum Berühren und Abnehmen einer biologischen Probe, wobei das Abtupfende (32) aus polymerem Schaum gefertigt ist und weicher sowie elastischer ist als der Handgriff,

**dadurch gekennzeichnet,** daß

der polymere Schaum des Abtupfendes (32) ein geschlossenzelliger polymerer Schaum ist und das Abtupfende eine Einrichtung mit diskontinuierlicher Oberfläche zur Erleichterung des Sammelns biologischer Proben aufweist.

2. Tupfer zum Sammeln von biologischen Proben nach Anspruch 1, bei dem das Abtupfende (32) steril ist.

3. Tupfer zum Sammeln von biologischen Proben nach Anspruch 1 oder 2, bei dem die Einrichtung mit diskontinuierlicher Oberfläche mindestens eine konkave Nut (34) in der Oberfläche des Abtupfendes (32) aufweist.

4. Tupfer zum Sammeln von biologischen Proben nach Anspruch 1 oder 2, bei dem die Einrichtung mit diskontinuierlicher Oberfläche mindestens eine von der Oberfläche des Abtupfendes (32) auswärts vorstehende Ausstülpung (52) aufweist.

5. Tupfer zum Sammeln von biologischen Proben nach einem der Ansprüche 1-4, bei dem der Griffteil (29) fester ist als der Schaftteil (31).

6. Tupfer zum Sammeln von biologischen Proben nach einem der Ansprüche 1-5, bei dem der Schaftteil (31) krummlinig geformt ist.

7. Tupfer zum Sammeln von biologischen Proben nach einem der Ansprüche 1-6, bei dem der Griffteil (29) krummlinig geformt ist.

8. Tupfer zum Sammeln von biologischen Proben nach einem der Ansprüche 1-5, bei dem der Schaftteil (31) krummlinig geformt ist und der Griffteil (29) krummlinig geformt ist, wobei der Ausgangspunkt des Krümmungsradius (RS) des Schaftteils (31) dem Ausgangspunkt des Krümmungsradius (RG) des Griffteils (29) entgegengesetzt ist.

9. Tupfer zum Sammeln von biologischen Proben nach Anspruch 1, bei dem das Abtupfende (32) aus geschlossenzelligem Polyethylenschaum gefertigt ist.

10. Tupfer zum Sammeln von biologischen Proben nach Anspruch 9, bei dem die Dichte des Schaums im wesentlichen etwa zwei Pfund pro Kubikfuß (32 Kilogramm pro Kubikmeter) beträgt.

**Revendications**

1. Un tampon pour prélever des échantillons biologiques, comprenant :

un manche allongé (21) muni d'une partie proximale (22) comprenant une extrémité proximale (23) et d'une partie distale (25) comprenant une extrémité distale (27), ledit manche comprenant une partie formant corps de manche (31) dans ladite partie distale (25), et une partie de préhension (29) dans ladite partie proximale (22) ; et

un embout de tampon (32) sur ladite extrémité distale (27) pour venir en contact sur un échantillon biologique et le prélever, ledit embout (32) étant réalisé en mousse de polymère, ledit embout étant plus mou et plus élastique que ledit manche, caractérisé en ce que ladite mousse de polymère dudit embout (32) est une mousse de polymère à cellules fermées et que ledit embout comprend des moyens de discontinuité de la surface pour faciliter le prélèvement de l'échantillon biologique.

2. Le tampon pour prélever des échantillons biologiques selon la revendication 1, dans lequel ledit embout (32) est stérile.

3. Le tampon pour prélever des échantillons biologiques selon la revendication 1 ou 2, dans lequel lesdits moyens de discontinuité de la surface comprennent au moins une rainure concave (34) ménagée dans la surface dudit embout (32).

4. Le tampon pour prélever des échantillons biologiques selon la revendication 1 ou 2, dans lequel lesdits moyens de discontinuité de la surface comprennent au moins une saillie (52) dirigée vers l'extérieur à partir de la surface dudit embout (32).

5. Le tampon pour prélever des échantillons biologiques selon l'une des revendications 1 à 4, dans lequel ladite partie de préhension (29) est plus rigide que ladite partie formant corps de manche (31).

6. Le tampon pour prélever des échantillons biologiques selon l'une des revendications 1 à 5, dans lequel ladite partie formant corps de manche (31) est de forme curviligne.

7. Le tampon pour prélever des échantillons biologiques selon l'une des revendications 1 à 6, dans lequel ladite partie de préhension (29) est de forme curviligne.

8. Le tampon pour prélever des échantillons biologiques selon l'une des revendications 1 à 5, dans lequel ladite partie formant corps de manche (31) est de forme curviligne et ladite partie de préhension (29) est de forme curviligne, de manière que l'origine du rayon de courbure (RS) de ladite partie formant corps de manche (31) soit opposée à l'origine du rayon de courbure (RG) de ladite partie de préhension (29).

9. Le tampon pour prélever des échantillons biologiques selon la revendication 1, dans lequel ledit embout (32) est réalisé à partir d'une mousse de polyéthylène à cellules fermées.

10. Le tampon pour prélever des échantillons biologiques selon la revendication 9, dans lequel ladite mousse a une densité sensiblement de 32 kilogrammes par mètre cube.

FIG. 1

FIG. 2

FIG.3

FIG. 4

FIG. 5

FIG. 6

FIG. 7

FIG. 8    FIG. 9